# EUROPEAN PATENT APPLICATION

(11) **EP 3 797 766 A1**
(43) Date of publication of application: **31.03.2021**
(21) Application number: 19199203.1
(22) Date of filing: 24.09.2019
(51) Int. Cl.: A61K 31/19, A61P 29/00

(54) **COMPOSITIONS FOR USE IN REDUCING INFLAMMATION**

(71) Applicant: Evonik Operations GmbH, 45128 Essen (DE)
(72) Inventor: ZETTL, Lisa., 64293 Darmstadt (DE); HEADLEY, Laura., 64367 Mühltal (DE); ENGEL, Andrea., 60599 Frankfurt (DE); BENEDIKT, Anne., 60431 Frankfurt (DE); KUHN, Ann-Katrin., 64293 Darmstadt (DE)
(74) Representative: Evonik Patent Association

(57) **Abstract**

The present invention is related to a composition for use in reducing inflammation, wherein the composition comprises at least one keto acid and/or a salt thereof, preferably selected from the branched chain keto acids keto leucine, keto valine and keto isoleucine.

## Description

The present invention is related to compositions for use in reducing inflammation, wherein the composition comprises at least one keto acid and/or a salt thereof, preferably selected from the branched chain keto acids keto leucine, keto valine and keto isoleucine.
Keto acids have differing functions in metabolism. The keto acid analogues of branched-chain amino acids play an important role in amino acid metabolism, especially in skeletal muscle and in the liver. One third of muscle protein consists of the branched-chain amino acids which cannot be formed by the body but must be taken in with the diet. In the muscle, particularly in the case of physical exertion, proteins are continuously broken down and re-synthesized, which results in the formation of the corresponding keto acid. The resultant keto acid can then be further oxidized enzymatically for energy production. The carrier is transported to the liver and there liberates ammonia which is converted into urea and excreted via the kidneys.
Branched-chain amino acids (BCAAs) are essential amino acids which are important components of proteins in human skeletal muscles. They are also known to modulate glucose metabolism and to support correct brain function. Thus, they are commonly used as nutritional supplements to improve mental and physical performance including the purpose of muscle building and maintaining muscle health.
The use of keto acids which are derived from branched-chain amino acids for pharmaceutical purposes has long been known (Wu et al. (2017), PLoS One, 12 (5), for patients with anemic advanced chronic kidney disease). For instance, keto isocaproate (= keto leucine) can be used for reducing the protein breakdown in muscle and for a reduction of the formation of urea resulting from protein breakdown after muscle operations (US 4,677,121). The use of keto leucine in malnutrition, muscular dystrophy or uremia and in other disorders which are a secondary consequence of protein breakdown in muscle is also described there. Keto leucine is administered in this case intravenously. In addition, it has been proposed to administer the keto acids of leucine, isoleucine and valine to patients who must maintain a protein-reduced diet, for example because of renal failure (US 4,100,161). The role of keto acids within protein metabolism with respect to various medical indications is also described in Walser, M. et al., Kidney International, Vol. 38 (1990), pp. 595-604. However, studies revealed that elevated BCAA levels can promote an inflammatory response in peripheral blood mononuclear cells and generate inflammation as well as oxidative stress in endothelial cells. In a recent mouse study, it was shown that BCAAs induced pro-inflammatory gene expression (Mu et al. (2018), nutrients, 10 (918)). Further studies revealed that elevated BCAA levels generate inflammation and oxidative stress in endothelial cells, thereby facilitating inflammatory cells adhesion and endothelial dysfunction (Zhenyukh et al. (2018), J Cell Mol Med., 22 (4948-4962)).
Besides BCAAs, branched-chain keto acids (BCKAs) can be used as an alternative to increase muscle protein synthesis rates and support as nutritional supplements (Shimomura, Y. et al., American Society for Nutrition). The use of keto acids of leucine, isoleucine and valine for improving muscle performance and also for supporting muscle recovery after fatigue is described in US 6,100,287, wherein salts of the corresponding anionic keto acids with cationic amino acids as counterion, such as, for example, arginine or lysine, are used. WO 2008/122473 A2 describes food supplements containing keto analogues of branched-chain amino acids for supporting muscle structure, increasing muscle performance and improving general wellbeing with simultaneous relief of nitrogen detoxification metabolism by reduced nitrogen supply compared to the intake of the corresponding amino acids and an improved nitrogen metabolism in the body.
WO 2010/112362 A1 discloses food supplements containing keto acids for supporting therapy in diabetes mellitus, in particular of type II. As a result, however, polyamines are also formed of which it is known that they can lead to apoptosis (programmed cell death). The excretion of the breakdown products of polyamines proceeds via the kidneys which are further stressed as a result.
In this context *in vitro* cell culture assays surprisingly revealed, that BCKAs as mixture as well as single keto acids could mediate an anti-inflammatory effect illustrated by reduced secretion of inflammatory cytokines by macrophages, in contrast to BCAAs, which showed pro-inflammatory tendencies. Thus, the present invention opens a broad range of anti-inflammatory applications in the field of nutrition, pharma and cell culture; BCKAs e.g. could be a solution for diseases connected to inflammatory processes as sarcopenia as well as diseases based on chronic inflammation and further detrimental pathophysiological changes to the body.

A "branched chain amino acid" (BCAA), according to the present invention, is understood as being an amino acid having an aliphatic side-chain with a branch (a central carbon atom bound to three or more carbon atoms). Among the proteinogenic amino acids, there are three BCAA: leucine, isoleucine, and valine. Non-proteinogenic BCAAs include 2-aminoisobutyric acid.
A "keto acid", according to the present invention, is understood as being an organic compound that contains a carboxylic acid group and a ketone group. The keto acids according to the invention are chosen from keto analogous of amino acids, namely keto leucine, keto valine, keto isoleucine and/or a salt of these keto acids. Salts of keto acids shall also include mixed salts of such keto acids. Such mixed salts can be produced by co-crystallization of different keto acids.
A "branched chain keto acid" (BCKA), according to the present invention, shall be understood as the keto analogous of the BCAAs, namely keto leucine, keto valine and keto isoleucine and/or salts of these BCKAs. Salts of BCKAs shall also include mixed salts of such BCKAs. Such mixed salts can be produced by co-crystallization of different BCKAs.

The present invention is related to a composition for use in reducing inflammation, wherein the composition comprises at least one keto acid and/or a salt thereof. The keto acids are preferably selected from the branched chain keto acids keto leucine, keto valine and keto isoleucine.
In a particularly preferred configuration, the composition comprises keto leucine, keto valine and keto isoleucine in an approximate ratio of 2:1:1.

In a preferred configuration, the composition comprises alkali metal salts or alkaline earth metal salts of the said keto acids. The alkaline earth metal is preferably chosen from magnesium and calcium, preferably calcium.
It is particularly preferred, when the alkaline earth metal salt is chosen from calcium carbonate, calcium hydroxide, calcium acetate, calcium chloride, calcium oxide, magnesium hydroxide and magnesium acetate.
In a preferred configuration of the present invention, the composition comprises at least two keto acids, preferably prepared by a process wherein two or more free keto acids or salts of keto acids are mixed, and the mixed keto acids are then co-crystallized with one or more alkaline earth metal salts.
It was a surprising finding that after co-crystallization of two or more keto acids no additional processing is necessary to achieve homogeneity with this product. The mixture obtained has a uniform particle size distribution and a homogenous crystal form.

The composition for use according to the present invention is preferably used for reducing and / or inhibiting the release of one or more inflammatory factors selected from the group consisting of interleukin 1β (IL-1β), interleukin 6 (IL-6) and tumor necrosis factor α (TNF-α).
For example, in a preferred configuration, the composition is used for the prevention and/or treatment of inflammation instigated by at least one of the aforementioned inflammatory factors occurring in any area of the body but particularly in connection with the development and progression of Cardiovascular Disease (including pathophysiological changes leading to endothelial dysfunction and atherosclerosis) and subsequent consequences to health (such as myocardial infarction or stroke). Additionally, the following detrimental pathophysiological changes to the body may benefit from the application of the present invention: the development and progression of insulin resistance, type-2 diabetes, metabolic syndrome, hypertension, 'inflamm-aging' (a scenario where there is a sustained, long-term and progressive increase in the proinflammatory status of an aging individual and is linked to increased disability and mortality) and post-exercise recovery (where inflammatory factors, such as TNF-α, increase in response to muscle damage).
In a further preferred configuration, the composition is used for treating or preventing a disease or disorder or as supportive medical nutrition, the disease or disorder being preferably selected from sarcopenia, multiple sclerosis (MS), rheumatoid arthritis (RA), renal disease, liver disease, psoriasis, psoriatic arthritis, colitis ulcerosa, Crohn's disease, myasthenia gravis (MG), autoimmune polyglandular syndrome type II (APS-II), Hashimoto's thyroiditis (HT), type-1 diabetes (T1D), systemic lupus erythematosus (SLE), autoimmune lymphoproliferative syndrome (ALS), Parkinson's disease, or an allergy, asthma, or an infectious disease.
The term "supportive medical nutrition" according to the present invention shall mean that the composition has supportive capacity in order to provide a degree of relief from symptoms of (but not intended to cure) a medical condition.

A food, drink, functional food or drink, food supplement, medical food, pharmaceutical product, preferably in form of a tablet or capsule or in powder form comprising the composition according to the present invention for use in the prevention and / or treatment of inflammation is also a subject of the present invention. In such a product, one single keto acid or a mixture of two or more keto acids and / or one or more keto acids in a processed form may be used.

A further subject of the present invention is directed to a cell culture medium comprising the composition according to the present invention for use in the prevention and / or treatment of inflammation in cultured cells.
In a specific embodiment, the culture medium comprises mixed salts of two or more keto acids. In this embodiment, two or more keto acids selected from keto leucine, keto valine, keto isoleucine are co-crystallized with one or more alkaline earth metal salts, preferably selected from calcium carbonate, calcium hydroxide, calcium acetate, calcium chloride, calcium oxide, magnesium hydroxide and magnesium acetate.
In a preferred configuration of the present invention the culture medium comprises keto leucine, keto valine and keto isoleucine in an approximate ratio of 2:1:1.
It is further preferred to use alkali metal salts or alkaline earth metal salts of the said keto acids.

### Examples

### Materials:

**Table 1: Materials used for cytokine release assay**

| **Material** | **Supplier** |
|---|---|
| THP-1 cells LOT 30 ATCC | DSMZ, Braunschweig (Germany) |
| Lipopolysaccharide (LPS) solution (500x) LOT 4351181 | Invitrogen, Carlsbad (USA) |
| Roswell Park Memorial Institute medium (RPMI 1640) | Gibco Life technologies, Carlsbad (USA) |
| Phorbol 12-myristate 13-acetate (PMA) Cat. No 360627-500G | Sigma Aldrich |
| *Fetal bovine serum (heat inactivated)* | Gibco Life technologies, Carlsbad (USA) |
| D (+) Glucose wasserfrei | Carl Roth, Karlsruhe (GER) |
| Gentamicin (50 mg/mL) 10 mL | Thermo Fisher Scientific GmbH, Dreieich (Germany) |
| Dimethylsulfoxid (DMSO) Cat. No A3672.0100 | VWR |
| CellTiter-Glo Luminescent Cell Viability Assay, (Cat. No G7570) | Promega GmbH, Madison (USA) |
| Human IL-6 ELISA kit (Cat. No RAB0306-1KT) | Sigma Aldrich, St. Louis (USA) |
| Human IL-1 β ELISA kit (Cat. No RAB0273-1KT) | Sigma Aldrich, St. Louis (USA) |
| Human TNF-α ELISA kit (Cat. No RAB0476-1KT) | Sigma Aldrich, St. Louis (USA) |
| Branched-chain keto acids (BCKA): Ca⁺-salts of keto leucine, keto isoleucine and keto valine in an approximate ratio of 2:1:1 | Evonik Nutrition & Care GmbH, Darmstadt (Germany) |
| Keto valine (Ca⁺-salt) | Evonik Nutrition & Care GmbH, Darmstadt (Germany) |
| Keto leucine (Ca⁺-salt) | Evonik Nutrition & Care GmbH, Darmstadt (Germany) |
| Keto isoleucine (Ca⁺-salt) | Evonik Nutrition & Care GmbH, Darmstadt (Germany) |

**Table 2: Devices used for cytokine release assay.**

| **Device** | **Supplier** |
|---|---|
| Safety cabinet HERA Safe 2020 | Thermo Fisher Scientific GmbH, Dreieich (Germany) |
| Automatic cell counter Countess | Thermo Fisher Scientific GmbH, Dreieich (Germany) |
| Centrifuge 5415R | Eppendorf, Hamburg (Germany) |
| Attune Nxt | Thermo Fisher Scientific GmbH, Dreieich (Germany) |
| Heating thermo shaker HTMR 133 | DITABIS Digital Biomedical Imaging Systems AG, Pforzheim (Germany) |
| Vortex-Genie 2 | Scientific Industries, Bohemia (USA) |
| TECAN Infinite 200 Pro | Tecan Group Ltd., Männedorf (Switzerland) |

### Methods:

### Cytokine release assay:

The assay used THP-1 cells, a monocyte suspension cell line, as starting point. In a first step, the monocytes were differentiated into macrophages via incubation with 50 nM PMA (phorbol 12-myristate 13-acetate) for 24 h followed by a resting period of 24 h in PMA free media in a CO2-Incubator (37°C, 5% CO₂, 95% humidity). Resulting macrophages adhere to the surface of cell culture plastic ware and could be used for the cytokine release assay.

On the first day of the assay, 7.5x10⁵ cells per well were seeded in a 6-well plate and differentiated into macrophages as described. During the resting time of 24 hours without PMA, either branched-chain keto acids (BCKA: Ca⁺-salts of keto leucine, keto isoleucine and keto valine in an approximate ratio of 2:1:1) or three different single keto acids (Ca⁺-salts of keto valine, keto leucine or keto isoleucine) were added in a concentration of 100 µg/mL to investigate its influence on cytokine release and immune reaction. This scenario should simulate the preventative consumption of the product (addition of BCKA prior to the LPS stimulus). The control was rested in medium without BCKA. After the resting period, immune reaction was triggered by addition of 100 ng/mL LPS. For treatment constantly to the inflammation, 100 µg/ml BCKA were added additionally to the LPS stimulation. After 24 hours of LPS exposure, cell culture medium was removed and prepared for cytokine measurement by ELISA.

1 mL of cell culture medium was used and centrifuged for 10 min at 1000xg. Cell culture supernatants were stored at -20°C until further usage for cytokine measurement. Single cytokine ELISAs for IL-6, IL-1β and TNF-α were used for the quantification of the respective cytokines in the cell culture supernatant. Absorbance was measured with a multiplate reader at 450 nm. Resulting cytokine amount was normalized to the number of cells in the sample after the assay procedure.

### Example 1: Effects of BCKA on the secretion of cytokines

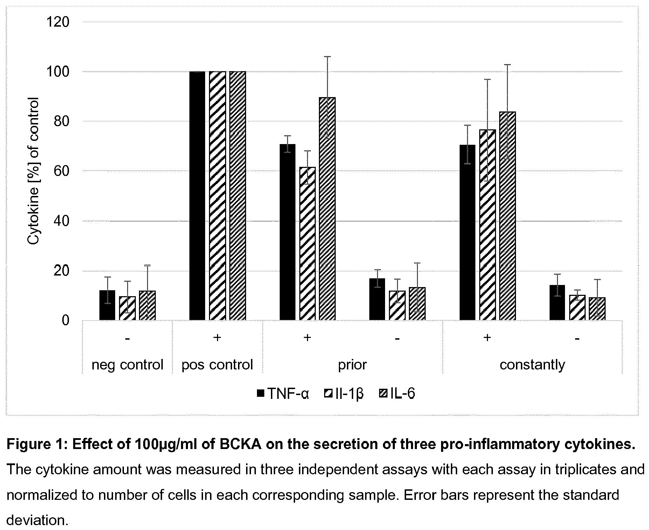

First, macrophages were obtained out of THP-1 monocytes via differentiation. When investigating the preventative scenario, BCKA was added 24 hours before inducing immune reaction through LPS. For the treatment with BCKA different time periods were chosen -prior and constantly. Treatment with BCKA stopped when LPS treatment was started (BCKA treatment prior to LPS stimulus) or treatment with BCKA was continued (BCKA treatment constantly to LPS stimulus). To evaluate the level of immune reaction, the pro-inflammatory cytokines TNF-α, IL-1β and IL-6 were measured in cell culture supernatants. The expression of pro-inflammatory cytokines of cells treated with BCKA is shown in Figure 1.

Figure 1 shows the effect of 100µg/ml of BCKA on the secretion of three pro-inflammatory cytokines. The cytokine amount was measured in three independent assays with each assay in triplicates and normalized to number of cells in each corresponding sample. Error bars represent the standard deviation.

Positive and negative control were not exposed to BCKA at any time and display the cytokine release triggered by lipopolysaccharides versus the stimulated baseline. Cells that were treated with BCKA and then with LPS as an inflammatory stimulus are marked as "(+) prior to inflammation" and cells that were not exposed to LPS but likewise with the BCKA"(-) prior to inflammation". The latter served as a control to check if BCKA itself caused cytokine release or interfered with the assay in another manner. This was excluded since cells that were treated with natural compounds showed the same cytokine profile as the negative control. In case of pretreatment with BCKA before the LPS stimulus, a significant reduction of the cytokine release could be observed. This confirms a significant effect of the prior and constantly treatment with BCKA on the immune reaction. Furthermore, similar results were obtained using the single keto acids keto-valine, keto-leucine and keto-isoleucine.

The expression of pro-inflammatory cytokines of cells treated with keto-valine is shown in Figure 2. Figure 3 shows the expression of pro-inflammatory cytokines treated with keto-leucine. In Figure 4 cells were treated with keto-isoleucine.

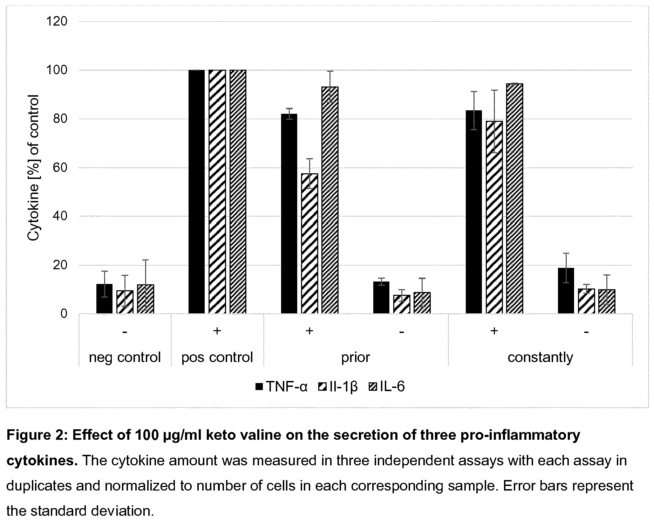

Figure 2 shows the effect of 100 µg/ml keto valine on the secretion of three pro-inflammatory cytokines. The cytokine amount was measured in three independent assays with each assay in duplicates and normalized to number of cells in each corresponding sample. Error bars represent the standard deviation.

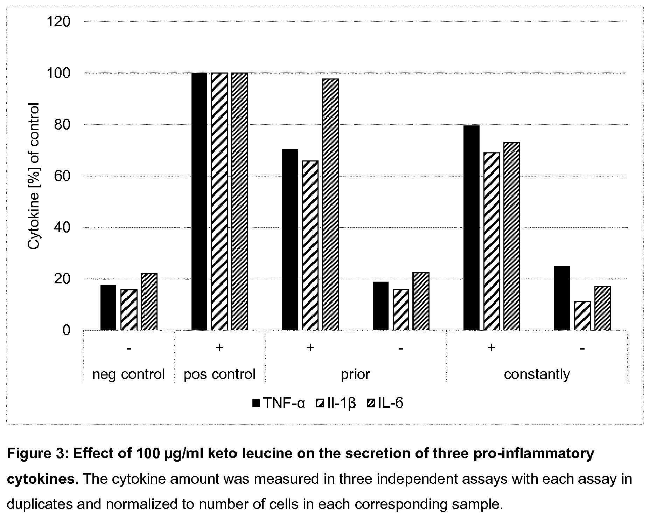

Figure 3 shows the effect of 100 µg/ml keto leucine on the secretion of three pro-inflammatory cytokines. The cytokine amount was measured in three independent assays with each assay in duplicates and normalized to number of cells in each corresponding sample.

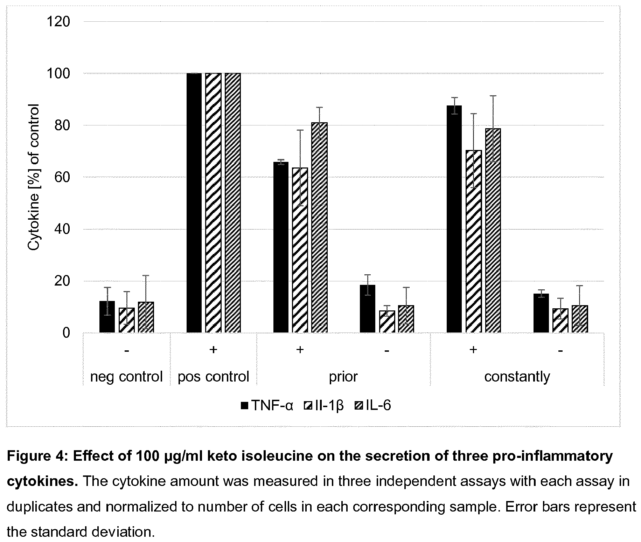

Figure 4: shows the effect of 100 µg/ml keto isoleucine on the secretion of three pro-inflammatory cytokines. The cytokine amount was measured in three independent assays with each assay in duplicates and normalized to number of cells in each corresponding sample. Error bars represent the standard deviation.

## Claims

1. A composition for use in reducing inflammation, wherein the composition comprises at least one keto acid and/or a salt thereof.

2. The composition for use according to claim 1, wherein the keto acids are selected from the branched chain keto acids keto leucine, keto valine and keto isoleucine.

3. The composition for use according to any of the preceding claims, wherein the composition comprises keto leucine, keto valine and keto isoleucine in an approximate ratio of 2:1:1.

4. The composition for use according to any of the preceding claims, comprising alkali metal salts or alkaline earth metal salts of the said keto acids.

5. The composition for use according to any of the preceding claims, wherein the alkaline earth metal is chosen from magnesium and calcium, preferably calcium.

6. The composition for use according to any of the preceding claims, comprising at least two keto acids, preferably prepared by a process wherein two or more free keto acids or salts of keto acids are mixed, and the mixed keto acids are then co-crystallized with one or more alkaline earth metal salts.

7. The composition for use according to any of the preceding claims, wherein the alkaline earth metal salt is chosen from calcium carbonate, calcium hydroxide, calcium acetate, calcium chloride, calcium oxide, magnesium hydroxide and magnesium acetate.

8. The composition for use according to any of the preceding claims, wherein the use is for reducing and / or inhibiting the release of one or more inflammatory factors selected from the group consisting of interleukin 1β (IL-1β), interleukin 6 (IL-6) and tumor necrosis factor α (TNF-α).

9. The composition for use according to any of the preceding claims, for treating or preventing detrimental pathophysiological changes in a subject, preferably selected from development and progression of cardiovascular disease, the development and progression of insulin resistance, type-2 diabetes, metabolic syndrome, hypertension, 'inflamm-aging' and post-exercise recovery.

10. The composition for use according to any one of claims 1 to 9, for treating or preventing a disease or disorder or as supportive medical nutrition, the disease or disorder being preferably selected from sarcopenia, multiple sclerosis (MS), rheumatoid arthritis (RA), renal disease, liver disease, psoriasis, psoriatic arthritis, colitis ulcerosa, Crohn's disease, myasthenia gravis (MG), autoimmune polyglandular syndrome type II (APS-II), Hashimoto's thyroiditis (HT), type-1 diabetes (T1D), systemic lupus erythematosus (SLE), autoimmune lymphoproliferative syndrome (ALS), Parkinson's disease, or an allergy, asthma, or an infectious disease.

11. A food, drink, functional food or drink, food supplement, medical food, pharmaceutical product, preferably in form of a tablet or capsule or in powder form comprising the composition of any one of claims 1 to 10 for use in the prevention and / or treatment of inflammation.

12. A composition according to claim 11, comprising one single keto acid or a mixture of two or more keto acids and / or one or more keto acids in a processed form.

13. A cell culture medium comprising the composition of any one of claims 1 to 8 for use in the prevention and / or treatment of inflammation.
